# FASCICULE DE BREVET EUROPEEN

(11) **EP 0 662 318 B1**
(45) Date de publication et mention de la délivrance du brevet: **28.08.1996**
(21) Numéro de dépôt: 94402692.1
(22) Date de dépôt: 24.11.1994
(51) Int. Cl.: A61K 7/48, A61K 31/60

(54) **Composition cosmétique et/ou dermatologique contenant des dérivés d'acide salicylique et procédé de solubilisation de ces dérivés**
Kosmetische und/oder dermatologische Zusammensetzung enthaltend Salicylsäurederivate sowie ihren Verfahren zum Auflösen
Cosmetic and/or dermatological composition containing salicylic acid derivatives and the solubilisation process of these derivatives

(30) Priorité: 10.01.1994 FR 9400175
(43) Date de publication de la demande: 12.07.1995
(73) Titulaire: L'OREAL, F-75008 Paris (FR)
(72) Inventeur: Griat, Jacqueline, F-94480 Ablon (FR); Picard, Elisabeth, F-78140 Velizy (FR); Laugier, Jean-Pierre, F-92160 Antony (FR)
(74) Mandataire: Lhoste, Catherine

(56) Documents cités:
- EP-A- 0 378 936
- EP-A- 0 570 230
- GB-A- 2 174 906

## Description

La présente invention se rapporte à un procédé de solubilisation de dérivés d'acide salicylique dans au moins une huile végétale, et aux compositions cosmétiques et/ou dermatologiques contenant un tel dérivé solubilisé, utilisables pour le traitement du corps et du visage, y compris du cuir chevelu et des ongles, et plus spécialement pour le traitement de l'acné et du vieillissement de la peau (rides, ridules, teint).

Ces compositions peuvent se présenter sous forme d'émulsions, de solutions huileuses ou de dispersions de vésicules lipidiques.

Il est connu d'utiliser des dérivés de l'acide salicylique comme agent kératolytique pour traiter l'acné et comme agent d'antivieillissement dans des compositions cosmétiques et/ou dermatologiques. Ainsi, les documents FR2581542 et EP378936 décrivent de tels dérivés.

Ces dérivés sont d'un grand intérêt, étant donné leurs effets biologiques sur la peau. Cependant, leur utilisation pose un problème dans la mesure où ils se présentent sous forme cristalline et où ils ne sont solubles ni dans l'eau ni dans les huiles traditionnellement utilisées dans le domaine cosmétique comme les huiles minérales (vaseline, paraffine).

Ainsi, si on les introduit tels quels dans les compositions cosmétiques et/ou dermatologiques, ils restent à l'état de cristaux, ce qui rend l'utilisation de la composition les contenant, inefficace pour le traitement de la peau.

Ces dérivés sont, par contre, solubles dans les alcools inférieurs comme l'éthanol ou l'isopropanol, ou dans des solvants tels que l'octyldodécanol, certains glycols, les alcools gras à chaîne courte (< C12) ou les esters à chaîne courte (< C12).

Les alcools inférieurs présentent l'inconvénient de dessécher et d'irriter la peau et on préfère donc éviter de les utiliser dans les produits de soin du corps et/ou du visage.

Les alcool gras et esters gras à chaîne courte et certains glycols permettant la solubilisation de ces dérivés entraînent la pénétration d'actifs, ce qui n'est pas forcément souhaitable dans des produits de soin.

Actuellement, on cherche de manière générale à limiter de plus en plus l'utilisation de solvants dans les produits de soin pour la peau, ces solvants n étant pas toujours bien tolérés et pouvant entraîner des irritations quand on les utilise en trop grande quantité.

La demanderesse a donc cherché à formuler les dérivés de l'acide salicylique dans des compositions contenant le moins possible de solvants.

Elle a donc trouvé de façon inattendue que les huiles végétales solubilisaient les dérivés de l'acide salicylique et permettaient leur introduction dans les compositions cosmétiques et/ou dermatologiques, sans que se produise de recristallisation de ces dérivés et sans qu'il soit nécessaire d'utiliser une grande quantité de solvant.

Certes, le document EP-A-570230 décrit des compositions contenant un dérivé d'acide salicylique et une huile végétale, mais l'huile végétale est alors introduite parmi d'autres constituants huileux et en une quantité nettement insuffisante pour permettre la solubilisation du dérivé d'acide salicylique.

Or, la demanderesse a trouvé, de manière surprenante, que seules les huiles végétales permettaient de solubiliser les dérivés d'acide salicylique, les autres huiles, et en particulier les huiles minérales, ne permettant pas d'obtenir un tel résultat, et que ce résultat ne pouvait être atteint que lorsque l'huile végétale est présente en une quantité au moins cinq fois supérieure à celle du dérivé d'acide salicylique.

L'invention a donc pour objet un procédé de solubilisation d'un dérivé d'acide salicylique, caractérisé en ce qu'il consiste à utiliser une huile végétale, le rapport en poids huile végétale/dérivé d'acide salicylique étant supérieur à 5.

Le rapport huile végétale/dérivé d'acide salicylique est de préférence supérieur à 10 et inférieur à 99.

En particulier, le dérivé de l'acide salicylique présente la formule (I) : 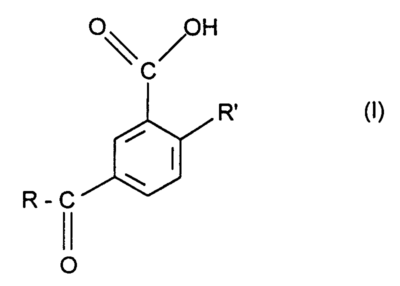
dans laquelle :
R représente une chaîne aliphatique saturée ayant de 3 à 11 atomes de carbone, linéaire, ramifiée ou cyclisée, une chaîne insaturée ayant de 3 à 17 atomes de carbone, portant une ou plusieurs doubles liaisons conjuguées ou non, les chaînes pouvant être substituées par au moins un substituant choisi parmi les atomes d'halogène, les groupements trifluorométhyle, les groupements hydroxyle sous forme libre ou estérifiée par un acide ayant de 1 à 6 atomes de carbone ou bien par une fonction carboxyle, libre ou estérifiée par un alcool inférieur ayant de 1 à 6 atomes de carbone ;
R' représente un groupement hydroxyle ou une fonction ester de formule : 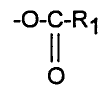
où R₁ est un groupement aliphatique, saturé ou insaturé ayant de 1 à 18 atomes de carbone.

L'invention a aussi pour objet une composition cosmétique et/ou dermatologique contenant un dérivé d'acide salicylique, de préférence de formule (I), solubilisé dans au moins une huile végétale, le rapport en poids huile végétale/dérivé d'acide salicylique étant supérieur à 5.

Comme dérivé de formule (I), on peut citer l'acide n-octanoyl-5-salicylique et l'acide n-dodécanoyl-5-salicylique.

Comme huiles végétales utilisables dans l'invention, on peut citer les huiles obtenues à partir de fleurs, de fruits ou de feuilles végétales, et notamment l'huile de tournesol, l'huile hybride de tournesol, l'huile de germes de blé, l'huile de jojoba, l'huile de maïs, l'huile de sésame, l'huile d'avocat, l'huile de coriandre, l'huile de carthame, l'huile hybride de carthame, l'huile de passiflore, l'huile d'olive, l'huile d'amande d'abricot, l'huile de macadamia, l'huile de rosa mosqueta, l'huile de pépins de fruits (raisin, cassis, orange, kiwi), l'huile de lin, l'huile d'onagre, l'huile de son de riz, l'huile d'arachide, l'huile de noisette, l'huile de pistache, l'huile de palme, l'huile de chia, l'huile de karité et leurs mélanges.

Selon une forme préférée de réalisation de l'invention, on utilise une huile végétale ayant un taux en acide palmitique supérieur à 5 % en poids (par rapport au poids total de la composition) et/ou un taux en acide oléique supérieur à 10 %, car une telle huile présente une meilleure stabilité à la chaleur vis-à-vis de l'oxydation. On peut citer comme huile de ce type l'huile hybride de tournesol, l'huile de palme et l'huile d'amande d'abricot.

Le dérivé d'acide salicylique est solubilisé dans une huile végétale ou un mélange d'huiles végétales, par exemple à chaud, à une température d'environ 60 °C à 100 °C, et mieux de 80 °C à 85 °C. La solution obtenue peut alors être introduite dans une composition cosmétique et/ou dermatologique qui peut être une dispersion de vésicules lipidiques ; une émulsion huile dans eau (H/E) ou eau dans huile (E/H) ; une solution huileuse ; l'émulsion ou la solution contenant éventuellement des vésicules lipidiques.

La quantité de dérivé d'acide salicylique que l'on peut dissoudre dépend de l'huile végétale utilisée et de la quantité de cette huile végétale. Elle est de préférence choisie de 0,01 % à 10 % en poids par rapport au poids total de la composition.

Selon la quantité de dérivé d'acide salicylique utilisée et pour éviter une recristallisation éventuelle à long terme, on peut ajouter une faible quantité de solvant à la composition cosmétique et/ou dermatologique. Le taux de solvant ajouté est fonction de la quantité de dérivé d'acide salicylique introduite : le rapport dérivé d'acide salicylique/solvant peut aller de 1/2,5 à 1/10.

Lorsque la composition est une émulsion H/E ou E/H, cette dernière contient de façon classique un émulsionnant H/E ou E/H.

Comme émulsionnants H/E, on peut citer notamment (CTFA) : le cétéarylglucoside vendu sous le nom de Montanov 68 par la Société SEPPIC, le PEG-40 stéarate vendu sous le nom de Myrj 52 par la société ICI, le sorbitan tristéarate vendu sous le nom de Span 65 par la société ICI, le sorbitan stéarate vendu sous le nom de Span 60 par la société ICI, le polysorbate 60 vendu sous le nom de Tween 60 par la société ICI, le mélange sorbitan stéarate/sucrose cocoate vendu sous le nom d'Arlatone 21-21 par la société ICI, le PPG-3 myristyl éther vendu sous le nom d'Emcol 249-3K par la société Witco qui peut aussi servir de solvant, le glycéryl stéarate, le PEG-20 stéarate et le PEG-100 stéarate.

Comme émulsionnant E/H, on peut citer notamment le mélange polyglycéryl-4 isostéarate/ cétyldiméthicone copolyol/ hexyl laurate vendu sous le nom d'Abil WE 09 par la société Goldschmidt.

Le taux d'émulsionnant peut aller de 0,1 % à 10 %, de préférence de 0,5 % à 5 %, en poids par rapport au poids total de la composition.

Dans les dispersions de vésicules lipidiques, l'émulsionnant est constitué par des vésicules de lipides ioniques (lécithine hydrogénée associée au stérol de soja oxyéthyléné) et/ou non ioniques.

Par ailleurs, les compositions de l'invention peuvent contenir dans la phase grasse, en plus du dérivé d'acide salicylique solubilisé dans la ou les huiles végétales, des huiles minérales (huile de vaseline), des huiles de synthèse, des huiles de silicone (cyclométhicone), des huiles perfluorées (perfluoropolyéthers), des alcools gras (alcool stéarylique, alcool cétylique) et des acides gras (acide stéarique).

Lorsque la composition se présente sous forme d'une solution huileuse, les huiles non végétales sont utilisées à raison de 5 % à 95 % du poids total de la composition.

Dans les émulsions E/H, la phase grasse représente de façon classique de 20 % à 70 % en poids par rapport au poids total de la composition. Dans les émulsions H/E, la phase grasse représente de façon classique de 5 % à 40 % en poids par rapport au poids total de la composition.

D'autre part les compositions de l'invention peuvent contenir les adjuvants habituels dans le domaine cosmétique, tels que les conservateurs, les parfums, les gélifants hydrophiles ou lipophiles, les matières colorantes et les actifs hydrophiles ou lipophiles. Les quantités utilisées de ces différents adjuvants sont celles classiquement utilisées dans le domaine cosmétique et/ou dermatologique, et par exemple de 0,01 % à 10 % du poids total de la composition. Ces adjuvants, selon leur nature, peuvent être introduits dans la phase grasse, dans la phase aqueuse et/ou dans les vésicules lipidiques.

Comme actifs hydrophiles, on peut utiliser les protéines ou les hydrolysats de protéine, les acides aminés, les polyols, en particulier la glycérine ou le sorbitol, l'urée, les alpha-hydroxy-acides, l'acide salicylique, les sucres et les dérivés de sucre.

Comme actifs lipophiles, on peut utiliser le rétinol (vitamine A) et ses dérivés, le tocophérol (vitamine E) et ses dérivés, les acides gras essentiels, les céramides et les huiles essentielles.

De façon avantageuse, on choisit des adjuvants diminuant l'effet irritant des dérivés d'acide salicylique. A cet effet, on utilise en particulier les acides gras essentiels tels que l'acide linoléique et l'acide linolénique, les céramides tels que l'oléoyldihydrosphingosine, les glycocéramides et les pseudocéramides. Par ailleurs, ces adjuvants empêchent la recristallisation des dérivés d'acide salicylique, et donc améliorent leurs solubilisations.

Comme gélifiants hydrophiles, on peut citer la gomme de xanthane, les polymères carboxyvinyliques, les dérivés de cellulose, les pectines, les polyacrylamides, en particulier le mélange polyacrylamide/C13-14 isoparaffine/laureth-7 vendu sous le nom Sepigel 305 par la Société SEPPIC.

On peut aussi utiliser dans ces compositions des filtres UV à propriété lipophile ou hydrophile, et les oxydes de titane ou de zinc.

L'invention se rapporte aussi à l'utilisation de la composition définie ci-dessus pour le traitement cosmétique de l'acné et du vieillissement et à un procédé de traitement cosmétique de l'acné et du vieillissement consistant à appliquer cette même composition sur la peau.

Les exemples ci-après de compositions selon l'invention, sont donnés à titre d'illustration et sans caractère limitatif. Les quantités y sont données en % en poids.

### Exemple 1 : Emulsion E/H

### Phase A :

| | |
|---|---|
| Polyglycéryl-4 isostéarate/cétyldiméthicone copolyol/hexyl laurate (Abil WE 09) | 4,00 |
| Heptaméthylnonane | 5,00 |
| Huile de tournesol | 9,10 |
| Huile de rosa mosqueta | 0,59 |
| Huile de pépins de cassis | 0,30 |
| Cyclométhicone | 3,50 |
| Acide n-octanoyl-5-salicylique | 1,00 |
| Parfum | 0,15 |
| Conservateur (propylparabène) | 0,10 |

### Phase B :

| | |
|---|---|
| Sulfate de magnésium | 0,65 |
| Glycérine | 10,00 |
| Eau | 62,65 |

### Phase C :

| | |
|---|---|
| Gomme de cellulose | 0,50 |
| Conservateur (quaternium-15) | 0,10 |
| Eau | qsp 100 % |

### Mode opératoire :

On chauffe les constituants de la phase A à 80°C jusqu'à dissolution complète des différents constituants. On refroidit à 65°C. On chauffe ensuite la phase B à 65°C, on la verse dans la phase A sous agitation ; on ajoute la phase C, puis on refroidit à 25°C.

La composition obtenue est une crème de soin ayant de bonnes propriétés nourrissantes et hydratantes.

### Exemple 2 : Emulsion H/E

### Phase A :

| | |
|---|---|
| Cétéarylglucoside (Montanov 68) | 0,18 |
| Acide n-octanoyl-5-salicylique | 1,5 |
| Huile d'amande d'abricot | 15,5 |
| Alcool stéarylique | 2,6 |
| Alcool cétylique | 0,7 |
| Acide stéarique | 1,0 |
| Octylméthoxy cinnamate (filtre) | 1,0 |
| Conservateur (butylparabène) | 0,15 |
| Octyldodécanol | 5,0 |
| Cyclométhicone | 5,0 |

### Phase B :

| | |
|---|---|
| Glycérine | 3,0 |
| Conservateur (méthylparabène) | 0,2 |
| Eau | 50,0 |

### Phase C :

| | |
|---|---|
| Polyacrylamide/C13-14 Isoparaffine/laureth-7 (Sepigel 305) | 0,6 |

### Phase D :

| | |
|---|---|
| Eau | qsp 100 % |

### Mode opératoire :

On porte les constituants de la phase A et ceux de la phase B à 80°C. On refroidit à 70°C, puis on verse la phase A dans la phase B sous agitation. On ajoute la phase C puis la phase D à 60°C toujours sous agitation. On obtient une crème de soin pour le visage ayant des propriétés hydratantes et protectrices.

### Exemple 3 : Emulsion H/E

### Phase A :

| | |
|---|---|
| Acide n-octanoyl-5-salicylique | 1,0 |
| Oléoyldihydrosphingosine | 0,10 |
| Huile de tournesol | 15,0 |

### Phase B :

| | |
|---|---|
| Huile de pépins de cassis | 1,00 |
| Sorbitan tristéarate (Span 65) | 0,90 |
| PEG-40 stéarate (Myrj 52) | 2,00 |
| Alcool cétylique | 4,00 |
| Glycéryl stéarate (coémulsionnant) | 3,00 |
| Conservateur (propylparabène) | 0,10 |

### Phase C :

| | |
|---|---|
| Glycérine | 3,0 |
| Conservateur (méthylparabène) | 0,2 |
| Eau | qsp 100 % |

### Mode opératoire :

On chauffe la phase A à 80°C et la phase B à 85°C, puis on les mélange. On ajoute le mélange à la phase C sous agitation à 65°C. On refroidit jusqu'à la température ambiante. On obtient une crème de soin du corps hydratante et nourrissante.

### Exemple 4 : Emulsion H/E

### Phase A :

| | |
|---|---|
| Acide n-octanoyl-5-salicylique | 0,50 |
| Heptaméthylnonane | 5,00 |
| Huile de tournesol | 15,00 |
| Huile de rosa mosqueta | 0,65 |
| Alcool stéarylique | 0,50 |
| Conservateur (propylparabène) | 0,10 |

### Phase B :

| | |
|---|---|
| Polyacrylamide/C13-14 Isoparaffine/laureth-7 (Sepigel 305) | 1,30 |

### Phase C :

| | |
|---|---|
| Sorbitan stéarate/ sucrose cocoate (Arlatone 21-21) | 2,50 |
| Glycérine | 3,00 |
| Parfum | 0,20 |
| Conservateur | 0,20 |
| Eau | qsp 100 % |

### Mode opératoire :

On chauffe la phase A à 80°C. On la refroidit à 70°C , puis on la verse dans la phase C sous agitation. On ajoute la phase B à 60°C toujours sous agitation. On refroidit sous agitation. On obtient une crème fluide hydratante pour le visage.

### Exemple 5 : Huile de soin

| | |
|---|---|
| Huile de tournesol | 80,6 |
| Huile de rosa mosqueta | 5,3 |
| Huile de pépins de cassis | 2,7 |
| Cyclométhicone | 10,0 |
| Acide n-octanoyl-5-salicylique | 1,0 |
| Parfum | 0,3 |
| Oléoyldihydrosphingosine | 0,1 |

### Mode opératoire :

On chauffe le mélange d'huiles végétales, d'acide n-octanoyl-5-salicylique et d'oléoyldihydrosphingosine à 80°C ; on refroidit l'ensemble à 40°C puis on ajoute les autres constituants. L'huile obtenue est nourrissante et protectrice ; elle peut être utilisée pour le visage et/ou le corps.

### Exemple 6 : Emulsion H/E

### Phase A :

| | |
|---|---|
| Acide n-octanoyl-5-salicylique | 1,0 |
| Oléoyldihydrosphingosine | 0,1 |
| Huile d'amande d'abricot | 14,5 |
| Huile de karité | 7,0 |
| PPG-3 myristyl éther (Emcol 249-3K) | 5,0 |
| Conservateur (propylparabène) | 0,1 |
| Polysorbate 60 (Tween 60) | 2,5 |
| Sorbitan Stéarate (Span 60) | 2,5 |

### Phase B :

| | |
|---|---|
| Cyclométhicone | 4,0 |
| Gomme de xanthane | 0,2 |
| Polymère carboxyvinylique | 0,5 |

### Phase C :

| | |
|---|---|
| Triéthanolamine (neutralisant) | 0,5 |
| Eau | 2,0 |

### Phase D :

| | |
|---|---|
| Conservateur (méthylparabène) | 0,2 |
| Glycérine | 5,0 |
| Eau | qsp 100 % |

### Mode opératoire :

On fait fondre les constituants de la phase A à 85°C, puis on refroidit la phase A à 70°C et on y introduit les phases B puis C et D sous agitation. On refroidit jusqu'à la température ambiante. On obtient une crème de jour hydratante qui agit contre le vieillissement naturel de la peau.

### Exemple 7 : Crème de soin pour le visage sous forme de dispersion de liposomes

### Phase A :

| | |
|---|---|
| Stérol de soja polyoxyéthyléné | 0,8 |
| Lécithine hydrogénée | 1,2 |

### Phase B :

| | |
|---|---|
| Eau | 28,2 |

### Phase C :

| | |
|---|---|
| Huile d'amande d'abricot | 14,5 |
| Oléoyldihydrosphingosine | 0,1 |
| Acide n-octanoyl-5-salicylique | 0,5 |
| Parfum | 0,2 |

### Phase D :

| | |
|---|---|
| Glycérine/polymère carboxyvinylique/eau (67,5/1/31,5) | 5,0 |
| Conservateur | 0,5 |
| Polyacrylamide/C13-14 isoparaffine/laureth-7(Sepigel 305) | 2,0 |
| Eau | qsp 100 % |

### Mode opératoire :

On fait fondre les constituants de la phase A à 100°C ; on ajoute progressivement la phase B à 80°C sous agitation, puis on fait deux passages à l'homogénéisateur à haute pression pour former les vésicules. On fait chauffer à 80-85°C les constituants de la phase C jusqu'à solubilisation complète. On refroidit et on ajoute la phase C dans le mélange des phases A et B. On fait deux passages de l'ensemble à l'homogénéisateur à haute pression, puis on refroidit et on ajoute la phase D sous agitation.

On obtient une crème hydratante qui est efficace pour lutter contre le vieillissement naturel de la peau.

### Exemple 8 : Emulsion H/E

### Phase A :

| | |
|---|---|
| Acide n-octanoyl-5-salicylique | 1 |
| Huile de tournesol | 4,2 |
| Huile de rosa mosqueta | 0,41 |
| Huile de pépins de cassis | 0,21 |
| Cyclométhicone | 4 |
| Alcool stéarylique | 0,6 |
| Alcool cétylique | 0,6 |
| Acide stéarique | 0,6 |
| Conservateur | 0,15 |
| Octyldodécanol | 5,0 |
| PEG-20 stéarate (émulsionnant) | 1,2 |
| PEG-100 stéarate (émulsionnant) | 0,6 |
| Glycéryl stéarate (coémulsionnant) | 0,6 |

### Phase B :

| | |
|---|---|
| Glycérine | 4 |
| Polymère carboxyvinylique | 0,05 |
| Hydroxyde de sodium (neutralisant) | 0,024 |
| Eau | qsp 100 |

### Phase C :

| | |
|---|---|
| Polyacrylamide/C13-14 Isoparaffine/laureth-7 (Sepigel 305) | 0,9 |

### Mode opératoire :

On chauffe les constituants de la phase A à 80°C jusqu'à dissolution complète des différents constituants. On la refroidit à 65°C et on l'introduit dans la phase B chauffée à 65°C sous agitation ; on ajoute ensuite au mélange la phase C, puis on refroidit à 25°C en continuant d'agiter.

La composition obtenue est une crème de soin ayant de bonnes propriétés nourrissantes.

## Revendications

1. Procédé de solubilisation d'un dérivé d'acide salicylique, caractérisé en ce que l'on utilise au moins une huile végétale, le rapport en poids huile végétale/dérivé d'acide salicylique étant supérieur à 5.

2. Procédé de solubilisation selon la revendication 1, caractérisé en ce que le rapport en poids huile végétale/dérivé d'acide salicylique est supérieur à 10.

3. Procédé de solubilisation selon la revendication 1 ou 2, caractérisé en ce que le dérivé d'acide salicylique présente la formule (I) : 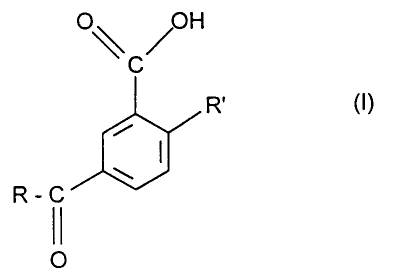 dans laquelle :
R représente une chaîne aliphatique saturée ayant de 3 à 11 atomes de carbone, linéaire, ramifiée ou cyclisée, une chaîne insaturée ayant de 3 à 17 atomes de carbone, portant une ou plusieurs doubles liaisons conjuguées ou non, les chaînes pouvant être substituées par au moins un substituant choisi parmi les atomes d'halogène, les groupements trifluorométhyle, les groupements hydroxyle sous forme libre ou estérifiée par un acide ayant de 1 à 6 atomes de carbone ou bien par une fonction carboxyle, libre ou estérifiée par un alcool inférieur ayant de 1 à 6 atomes de carbone ;
R' représente un groupement hydroxyle ou une fonction ester de formule : 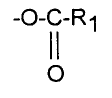 où R₁ est un groupement aliphatique saturé ou insaturé ayant de 1 à 18 atomes de carbone.

4. Procédé selon l'une quelconque des revendications 1 à 3, caractérisé en ce que l'huile végétale est au moins une huile choisie parmi l'huile de tournesol, l'huile hybride de tournesol, l'huile de germes de blé, l'huile de jojoba, l'huile de maïs, l'huile de sésame, l'huile d'avocat, l'huile de coriandre, l'huile de carthame, l'huile hybride de carthame, l'huile de passiflore, l'huile d'olive, l'huile d'amande d'abricot, l'huile de macadamia, l'huile de rosa mosqueta, l'huile de pépins de fruits, l'huile de lin, l'huile d'onagre, l'huile de son de riz, l'huile d'arachide, l'huile de noisette, l'huile de pistache, l'huile de palme, l'huile de chia, l'huile de karité et leurs mélanges.

5. Procédé selon l'une quelconque des revendications 1 à 4, caractérisé en ce que l'huile végétale est une huile présentant un taux de plus de 10 % en poids en acide oléique.

6. Procédé selon l'une quelconque des revendications 1 à 5, caractérisé en ce que l'huile végétale est une huile présentant un taux de plus de 5 % en poids en acide palmitique.

7. Procédé selon l'une quelconque des revendications 1 à 6, caractérisé en ce que le mélange est réalisé à chaud.

8. Procédé selon la revendication 7, caractérisé en ce que le mélange est réalisé à 60-100 °C.

9. Composition cosmétique et/ou dermatologique, caractérisée en ce qu'elle contient au moins un dérivé d'acide salicylique de formule ci-après, solubilisé dans au moins une huile végétale, le rapport en poids huile végétale/dérivé d'acide salicylique étant supérieur à 5 : 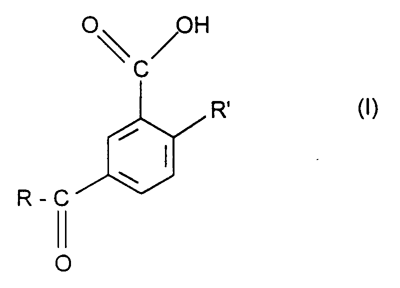 dans laquelle :
R représente une chaîne aliphatique saturée ayant de 3 à 11 atomes de carbone, linéaire, ramifiée ou cyclisée, une chaîne insaturée ayant de 3 à 17 atomes de carbone, portant une ou plusieurs doubles liaisons conjuguées ou non, les chaînes pouvant être substituées par au moins un substituant choisi parmi les atomes d'halogène, les groupements trifluorométhyle, les groupements hydroxyle sous forme libre ou estérifiée par un acide ayant de 1 à 6 atomes de carbone ou bien par une fonction carboxyle, libre ou estérifée par un alcool inférieur ayant de 1 à 6 atomes de carbone ;
R' représente un groupement hydroxyle ou une fonction ester de formule : 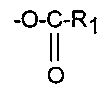 où R₁ est un groupement aliphatique saturé ou insaturé ayant de 1 à 18 atomes de carbone.

10. Composition cosmétique et/ou dermatologique selon la revendication 9, caractérisée en ce que le rapport en poids huile végétale/dérivé d'acide salicylique est supérieur à 10.

11. Composition cosmétique et/ou dermatologique selon la revendication 9 ou 10, caractérisée en ce que l'huile végétale est au moins une huile choisie parmi l'huile de tournesol, l'huile hybride de tournesol, l'huile de germes de blé, l'huile de jojoba, l'huile de maïs, l'huile de sésame, l'huile d'avocat, l'huile de coriandre, l'huile de carthame, l'huile hybride de carthame, l'huile de passiflore, l'huile d'olive, l'huile d'amande d'abricot, l'huile de macadamia, l'huile de rosa mosqueta, l'huile de pépins de fruits, l'huile de lin, l'huile d'onagre, l'huile de son de riz, l'huile d'arachide, l'huile de noisette, l'huile de pistache, l'huile de palme, l'huile de chia, l'huile de karité et leurs mélanges.

12. Composition cosmétique et/ou dermatologique selon l'une quelconque des revendications 9 à 11, caractérisée en ce que l'huile végétale est une huile présentant un taux de plus de 10 % en poids en acide oléique.

13. Composition cosmétique et/ou dermatologique selon l'une quelconque des revendications 9 à 12, caractérisée en ce que l'huile végétale est une huile présentant un taux de plus de 5 % en poids en acide palmitique.

14. Composition cosmétique et/ou dermatologiques selon l'une quelconque des revendications 9 à 13, caractérisée en ce qu'elle se présente sous forme d'émulsion H/E.

15. Composition cosmétique et/ou dermatologiques selon l'une quelconque des revendications 9 à 13, caractérisée en ce qu'elle se présente sous forme d'une solution huileuse.

16. Composition cosmétique et/ou dermatologique selon l'une quelconque des revendications 9 à 13, caractérisée en ce qu'elle se présente sous forme d'une dispersion de vésicules lipidiques.

17. Composition cosmétique et/ou dermatologique selon l'une quelconque des revendications 9 à 16, caractérisée en ce que le dérivé d'acide salicylique est l'acide n-octanoyl-5-salicylique.

18. Composition cosmétique et/ou dermatologique selon l'une quelconque des revendications 9 à 17, caractérisée en ce qu'elle contient, en outre, un adjuvant choisi parmi les céramides, les pseudocéramides, les glycocéramides et les acides gras essentiels.

19. Utilisation de la composition selon l'une quelconque des revendications 9 à 18 pour le traitement cosmétique de la peau contre l'acné et le vieillissement.

20. Procédé de traitement cosmétique contre l'acné et le vieillissement consistant à appliquer sur la peau la composition selon l'une quelconque des revendications 9 à 19.

## Claims

1. Process for the sobubilization of a salicylic acid derivative, characterized in that at least one vegetable oil is used, the ratio by weight between vegetable oil and salicylic acid derivative being greater than 5.

2. Solubilization process according to Claim 1, characterized in that the ratio by weight between vegetable oil and salicylic acid derivative is greater than 10.

3. Solubilization process according to Claim 1 or 2, characterized in that the salicylic acid derivative has the formula (I) : 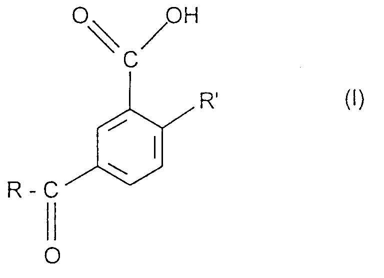 in which :
R represents a saturated linear, branched or cyclized aliphatic chain having from 3 to 11 carbon atoms, or an unsaturated chain having from 3 to 17 carbon atoms and bearing one or more conjugated or unconjugated double bonds, it being possible for the chains to be substituted with at least one substituent chosen from halogens atoms, trifluoromethyl groups and hydroxyl groups in free form or in a form esterified with an acid having from 1 to 6 carbon atoms, or alternatively with a carboxyl function, free or esterified with a lower alcohol having from 1 to 6 carbon atoms ;
R' represents a hydroxyl group or an ester function of formula : 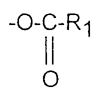 Where R₁ is a saturated or unsaturated aliphatic group having from 1 to 18 carbon atoms.

4. Process according to any one of Claims 1 to 3, characterized in that the vegetable oil is at least one oil chosen from sunflower oil, sunflower hybrid oil, wheat-germ oil, jojoba oil, maize oil, sesame oil, avocado oil, coriander oil, safflower oil, safflower hybrid oil, passion-flower oil, olive oil, apricot-kernel oil, macadamia oil, musk-rose oil, fruit-pip oil, linseed oil, evening-primrose oil, rice-bran oil, groundnut oil, hazelnut oil, pistachio oil, palm oil, chia oil, shea oil and mixtures thereof.

5. Process according to any one of Claims 1 to 4, characterized in that the vegetable oil is an oil having an oleic acid content of more than 10 % by weight.

6. Process according to any one of Claims 1 to 5, characterized in that the vegetable oil is an oil having a palmitic acid content of more than 5 % by weight.

7. Process according to any one of Claims 5 to 6, characterized in that the mixing is carried out in the heated state.

8. Process according to Claim 7, characterized in that the mixing is carried out at 60 - 100 °C.

9. Cosmetic and/or dermatological composition, characterized in that it contains at least one salicylic acid derivative of the formula below, solubilized in at least one vegetable oil, the ratio by weight between vegetable oil and salicylic acid derivative being greater than 5 : 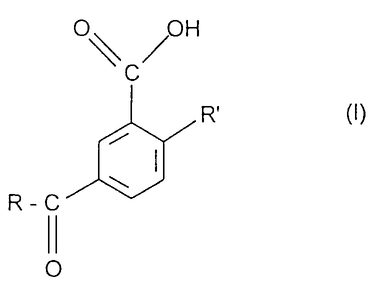 in which :
R represents a saturated linear, branched or cyclized aliphatic chain having from 3 to 11 carbon atoms, or an unsaturated chain having from 3 to 17 carbon atoms and bearing one or more conjugated or unconjugated double bonds, it being possible for the chains to be substituted with at least one substituent chosen form halogen atoms, trifluoromethyl groups and hydroxyl groups in free form or in a form esterified with an acid having from 1 to 6 carbon atoms, or alternatively with a carboxyl function, free or esterified with a lower alcohol having from 1 to 6 carbon atoms ;
R' represents a hydroxyl group or an ester function of formula : 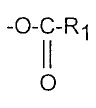 where R₁ is a saturated or unsaturated aliphatic group having from 1 to 18 carbon atoms.

10. Cosmetic and/or dermatological composition according to Claim 9, characterized in that the ratio by weight between vegetable oil and salicylic acid derivative is greater than 10.

11. Cosmetic and/or dermatological composition according to Claim 9 or 10, characterized in that the vegetable oil is at least one oil chosen from sunflower oil, sunflower hybrid oil, wheat-germ oil, jojoba oil, maize oil, sesame oil, avocado oil, coriander oil, safflower oil, safflower hybrid oil, passion-flower oil, olive oil, apricot-kernel oil, macadamia oil, musk-rose oil, fruit-pip oil, linseed oil, evening-primrose oil, rice-bran oil, groundnut oil, hazelnut oil, pistachio oil, palm oil, chia oil, shea oil and mixtures thereof.

12. Cosmetic and/or dermatological composition according to any one of Claims 9 to 11, charaterized in that the vegetable oil is an oil having an oleic acid content of more than 10 % by weight.

13. Cosmetic and/or dermatological composition according to any one of Claims 9 to 12, characterized in that the vegetable oil is an oil having a palmitic acid content of more than 5 % by weight.

14. Cosmetic and/or dermatological composition according to any one of Claims 9 to 13, characterized in that it takes the form of an O/W emulsion.

15. Cosmetic and/or dermatological composition according to any one of Claims 9 to 13, characterized in that it takes the form of an oily solution.

16. Cosmetic and/or dermatological composition according to any one of Claims 9 to 13, characterized in that it takes the form of a dispersion of lipid vesicles.

17. Cosmetic and/or dermatological composition according to any one of Claims 9 to 16, characterized in that the salicylic acid derivative is 5-n-octanoylsalicylic acid.

18. Cosmetic and/or dermatological composition according to any one of Claims 9 to 17, characterized in that it contains, in addition, an adjuvant chosen form ceramides, pseudoceramides, glycoceramides and essential fatty acids.

19. The use of the composition according to any of Claims 9 to 18, for cosmetic treatment of the skin against acne and ageing.

20. Process for cosmetic treatment against acne and ageing, which consists in applying to the skin the composition according to any one of Claims 9 to 18.

## Patentansprüche

1. Verfahren zur Solubilisation von Salicylsäurederivaten, dadurch gekennzeichnet, daß mindestens ein pflanzliches Öl verwendet wird, wobei das Gewichtsverhältnis pflanzliches Öl/Salicylsäurederivat größer als 5 ist.

2. Verfahren zur Solubilisation nach Anspruch 1, dadurch gekennzeichnet, daß das Gewichtsverhältnis pflanzliches Öl/Salicylsäurederivat größer als 10 ist.

3. Verfahren zur Solubilisation nach Anspruch 1 oder 2, dadurch gekennzeichnet, daß das Salicylsäurederivat die Formel (I) aufweist: 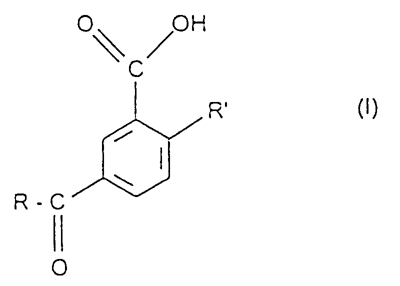 worin bedeuten:
R eine geradkettige, verzweigte oder cyclische aliphatische gesättigte Kette mit 3 bis 11 Kohlenstoffatomen, eine ungesättigte Kette mit 3 bis 17 Kohlenstoffatomen, die eine oder mehrere ggfs. konjugierte Doppelbindungen aufweist, wobei die Ketten mit mindestens einem Substituenten, der unter Halogenatomen, Trifluormethyl, Hydroxy in freier Form oder verestert mit einer Säure mit 1 bis 6 Kohlenstoffatomen ausgewählt ist, oder auch mit einer freien oder mit einem Alkohol mit weniger als 1 bis 6 Kohlenstoffatomen veresterten Carboxygruppe substituiert sein können, und
R' eine Hydroxygruppe oder eine Estergruppe der Formel: 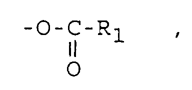 worin R₁ eine gesättigte oder ungesättigte aliphatische Gruppe mit 1 bis 18 Kohlenstoffatomen bedeutet.

4. Verfahren nach einem der Ansprüche 1 bis 3, dadurch gekennzeichnet, daß das pflanzliche Öl mindestens ein Öl ist, das ausgewählt ist unter Sonnenblumenöl, Öl aus Sonnenblumenhybriden, Weizenkeimöl, Jojobaöl, Maisöl, Sesamöl, Avocadoöl, Korianderöl, Safloröl, Öl aus Saflorhybriden, Öl aus Passionsblumen, Olivenöl, Aprikosenkernöl, Macadamiaöl, Öl aus rosa mosqueta, Öl aus Fruchtkernen, Leinöl, Nachtkerzenöl, Reiskleieöl, Erdnußöl, Haselnußöl, Pistazienöl, Palmöl, Chiaöl und Butterbaumöl sowie deren Gemischen.

5. Verfahren nach einem der Ansprüche 1 bis 4, dadurch gekennzeichnet, daß das pflanzliche Öl ein Öl ist, das einen Gehalt von mehr als 10 Gew.-% Ölsäure aufweist.

6. Verfahren nach einem der Ansprüche 1 bis 5, dadurch gekennzeichnet, daß das pflanzliche Öl ein Öl ist, das einen Gehalt von mehr als 5 Gew.-% Palmitinsäure aufweist.

7. Verfahren nach einem der Ansprüche 1 bis 6, dadurch gekennzeichnet, daß in der Wärme vermischt wird.

8. Verfahren nach Anspruch 7, dadurch gekennzeichnet, daß bei 60-100 °C vermischt wird.

9. Kosmetische und/oder dermatologische Zusammensetzung, dadurch gekennzeichnet, daß sie mindestens ein Salicylsäurederivat der nachfolgenden Formel enthält, das in mindestens einem pflanzlichen Öl gelöst ist, wobei das Gewichtsverhältnis pflanzliches Öl/Salicylsäurederivat größer als 5 ist: 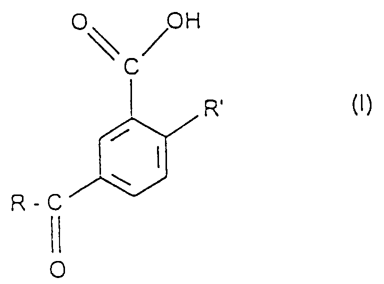 worin bedeuten:
R eine geradkettige, verzweigte oder cyclische aliphatische gesättigte Kette mit 3 bis 11 Kohlenstoffatomen, eine ungesättigte Kette mit 3 bis 17 Kohlenstoffatomen, die eine oder mehrere ggfs. konjugierte Doppelbindungen aufweist, wobei die Ketten mit mindestens einem Substituenten, der unter Halogenatomen, Trifluormethyl, Hydroxy in freier Form oder verestert mit einer Säure mit 1 bis 6 Kohlenstoffatomen ausgewählt ist, oder auch mit einer freien oder mit einem Alkohol mit weniger als 1 bis 6 Kohlenstoffatomen veresterten Carboxygruppe substituiert sein können, und
R' eine Hydroxygruppe oder eine Estergruppe der Formel: 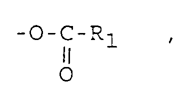 worin R₁ eine gesättigte oder ungesättigte aliphatische Gruppe mit 1 bis 18 Kohlenstoffatomen bedeutet.

10. Kosmetische und/oder dermatologische Zusammensetzung nach Anspruch 9, dadurch gekennzeichnet, daß das Gewichtsverhältnis pflanzliches Öl/Salicylsäurederivat größer als 10 ist.

11. Kosmetische und/oder dermatologische Zusammensetzung nach Anspruch 9 oder 10, dadurch gekennzeichnet, daß das pflanzliche Öl mindestens ein Öl ist, das ausgewählt ist unter Sonnenblumenöl, Öl aus Sonnenblumenhybriden, Weizenkeimöl, Jojobaöl, Maisöl, Sesamöl, Avocadoöl, Korianderöl, Safloröl, Öl aus Saflorhybriden, Öl aus Passionsblumen, Olivenöl, Öl aus Aprikosenkernen, Macadamiaöl, Öl aus rosa mosqueta, Öl aus Fruchtkernen, Leinöl, Nachtkerzenöl, Reiskleieöl, Erdnußöl, Haselnußöl, Pistazienöl, Palmöl, Chiaöl und Butterbaumöl sowie deren Gemischen.

12. Kosmetische und/oder dermatologische Zusammensetzung nach einem der Ansprüche 9 bis 11, dadurch gekennzeichnet, daß das pflanzliche Öl ein Öl ist, das einen Gehalt von mehr als 10 Gew.-% Ölsäure aufweist.

13. Kosmetische und/oder dermatologische Zusammensetzung nach einem der Ansprüche 9 bis 12, dadurch gekennzeichnet, daß das pflanzliche Öl ein Öl ist, das einen Gehalt von mehr als 5 Gew.-% Palmitinsäure aufweist.

14. Kosmetische und/oder dermatologische Zusammensetzung nach einem der Ansprüche 9 bis 13, dadurch gekennzeichnet, daß sie in Form einer O/W-Emulsion vorliegt.

15. Kosmetische und/oder dermatologische Zusammensetzung nach einem der Ansprüche 9 bis 13, dadurch gekennzeichnet, daß sie in Form einer Öllösung vorliegt.

16. Kosmetische und/oder dermatologische Zusammensetzung nach einem der Ansprüche 9 bis 13, dadurch gekennzeichnet, daß sie in Form einer Dispersion von Lipidvesikeln vorliegt.

17. Kosmetische und/oder dermatologische Zusammensetzung nach einem der Ansprüche 9 bis 16, dadurch gekennzeichnet, daß das Salicylsäurederivat 5-n-Octanoylsalicylsäure ist.

18. Kosmetische und/oder dermatologische Zusammensetzung nach einem der Ansprüche 9 bis 17, dadurch gekennzeichnet, daß sie ferner einen Zusatz enthält, der ausgewählt ist unter Ceramiden, Pseudoceramiden, Glycoceramiden und essentiellen Fettsäuren.

19. Verwendung der Zusammensetzung nach einem der Ansprüche 9 bis 18 zur kosmetischen Behandlung der Haut gegen Akne und Alterung.

20. Verfahren zur kosmetischen Behandlung gegen Akne und Alterung, das im Auftragen einer Zusammensetzung nach einem der Ansprüche 9 bis 19 auf die Haut besteht.
